# EUROPEAN PATENT APPLICATION

(11) **EP 4 455 634 A1**
(43) Date of publication of application: **30.10.2024**
(21) Application number: 23170762.1
(22) Date of filing: 28.04.2023
(51) Int. Cl.: G01N 15/14, G01N 33/53, G01N 33/533, G01N 33/536

(54) **ON-BOARD CONJUGATION**

(71) Applicant: F. Hoffmann-La Roche AG, 4070 Basel (CH); Roche Diagnostics GmbH, 68305 Mannheim (DE)
(72) Inventor: Hoogendijk, Jan-Gerrit, 6343 Rotkreuz ZG (CH)
(74) Representative: Altmann Stößel Dick Patentanwälte PartG mbB

(57) **Abstract**

The present invention relates to a method for labeling an analyte in a sample, said method comprising (a) providing a detection agent binding to the analyte and conjugating said detection agent with an indicator agent to produce a conjugation product; (b) contacting said sample with said conjugation product of step (a); wherein said step (b) is performed directly after completion of step (a) and wherein steps (a) and (b) are performed by the same labeling device; and to labeling devices, systems, and uses related thereto.

## Description

The present invention relates to a method for labeling an analyte in a sample, said method comprising (a) providing a detection agent binding to the analyte and conjugating said detection agent with an indicator agent to produce a conjugation product; (b) contacting said sample with said conjugation product of step (a); wherein said step (b) is performed directly after completion of step (a) and wherein steps (a) and (b) are performed by the same labeling device; the present invention also relates to labeling devices, systems, and uses related thereto.

Labeled antibodies have a wide variety of uses, in particular in clinical and diagnostic applications, but also in life science research. Many applications require only one labeled antibody; however, there are also more demanding applications in which more than one analyte needs to be detected, e.g. in multicolor FACS analyses of cell populations. Multicolor labeling frequently is hampered by interference between the labels, e.g. in fluorescence applications, fluorescence spillover, overlap of absorption spectra, and cross-excitation are well known interference phenomena.

Examples of multicolor labeling include 3 to 6 color panels used in HIV immunophenotyping, as e.g. reviewed by Barnett et al. (2009), Nat Rev Microbiol 6(11 Suppl):S7 and the 6 color TBNK panel manufactured by BioLegend; however, also more complex panels were used, e.g. in analysis of acute myeloid leukemia (Ouyang et al. (2019), J Int Med Res 47(4): 1483).

Labeled antibodies are usually provided by manufacturers, which means that each antibody/label combination must be obtained and stocked, which adds to storage and shipment costs. This effort increases exponentially with increasing numbers of antibodies and labels which may be used. Also, keeping a large number of antibody/label conjugates in stock may cause in particular rarely used combinations to become unusable, e.g. caused by on-board instability, open vial instability and/or or closed vial instability, i.e. for reaching the use-by date.

There is, thus, a need in the art for improved means and methods for provision and stockage of detection agent/label conjugates, avoiding the drawbacks as referred to above. The technical problem underlying the present invention can be seen as the provision of means and methods for complying with the aforementioned needs. The technical problem is solved by the embodiments characterized in the claims and herein below.

In accordance, the present invention relates to a method for labeling an analyte in a sample, said method comprising
(a) providing a detection agent binding to the analyte and conjugating said detection agent with an indicator agent to produce a conjugation product;
(b) contacting said sample with said conjugation product of step (a);
wherein said step (b) is performed directly after completion of step (a).

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" in an embodiment refer to "comprising one or more", i.e. are equivalent to "comprising at least one". In accordance, expressions relating to one item of a plurality, unless otherwise indicated, in an embodiment relate to at least one such item, in a further embodiment a plurality thereof; thus, e.g. identifying "a cell" relates to identifying at least one cell, in an embodiment to identifying a multitude of cells. Also, the term "multitude" is understood by the skilled person to relate to a number of more than one, i.e. in an embodiment at least two, in a further embodiment at least three, in a further embodiment at least four, in a further embodiment at least five; thus, a multitude may be e.g. 2, 3, 4, 5, 6, 7, 8, 9, 10, 15, 20, 25, 50, or 100.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

The methods specified herein below are in vitro methods. The method steps may, in principle, be performed in any arbitrary sequence deemed suitable by the skilled person, but in an embodiment are performed in the indicated sequence; also, one or more, in an embodiment all, of said steps may be assisted or performed by automated equipment. Moreover, the methods may comprise steps in addition to those explicitly mentioned above.

As used herein, the term "standard conditions", if not otherwise noted, relates to IUPAC standard ambient temperature and pressure (SATP) conditions, i.e. preferably, a temperature of 25°C and an absolute pressure of 100 kPa; also preferably, standard conditions include a pH of 7. Moreover, if not otherwise indicated, the term "about" relates to the indicated value with the commonly accepted technical precision in the relevant field, in an embodiment relates to the indicated value ± 20%, in a further embodiment ± 10%, in a further embodiment ± 5%. Further, the term "essentially" indicates that deviations having influence on the indicated result or use are absent, i.e. potential deviations do not cause the indicated result to deviate by more than ± 20%, in a further embodiment ± 10%, in a further embodiment ± 5%. Thus, "consisting essentially of' means including the components specified but excluding other components except for materials present as impurities, unavoidable materials present as a result of processes used to provide the components, and components added for a purpose other than achieving the technical effect of the invention. For example, a composition defined using the phrase "consisting essentially of' encompasses any known acceptable additive, excipient, diluent, carrier, and the like. In an embodiment, a composition consisting essentially of a set of components will comprise less than 5% by weight, in a further embodiment less than 3% by weight, in a further embodiment less than 1% by weight, in a further embodiment less than 0.1% by weight of non-specified component(s).

The term "fragment" of a biological macromolecule, in an embodiment of a polynucleotide or polypeptide, is used herein in a wide sense relating to any sub-part, in an embodiment subdomain, of the respective biological macromolecule comprising the indicated sequence, structure and/or function. Thus, the term includes sub-parts generated by actual fragmentation of a biological macromolecule, but also sub-parts derived from the respective biological macromolecule in an abstract manner, e.g. in silico. Thus, as used herein, an Fc or Fab fragment may be referred to as fragments of an immunoglobulin. The term "derivative" is understood by the skilled person to relate to a compound having a structure non-identical to the compound the derivative is derived from, but having essentially the same activity. Thus, a derivative of an antibody may in particular be a humanized antibody, a bispecific antibody, a single-chain antibody, or a nanobody, all of which are in principle known to the skilled person.

The term "labeling" is understood by the skilled person in the context of the instant description to relate to any activity and/or proceeding making an analyte detectable, in particular by causing the analyte to become associated with an indicator agent as specified herein below. In an embodiment, labeling is specific labeling, the term "specific labeling" relating to a labeling which enables differentiation between analyte and non-analyte molecules. As is understood by the skilled person, specific labeling does not necessarily have to enable differentiation between the analyte and any non-analyte molecule; typically, a sample will comprise a limited number of non-analyte compounds, in particular in case the sample is enriched for the analyte, is treated to remove non-analyte compound classes, e.g. polynucleotides and/or polypeptides, or is otherwise pretreated to reduce matrix complexity. Thus, as used herein, specific labeling in an embodiment is specific in the context of a particular sample type and/or sample pre-treatment. In an embodiment, specific labeling is labeling of the analyte more intensely by a factor of at least 5, in a further embodiment at least 10, in a further embodiment at least 25, in a further embodiment at least 100, compared to any non-analyte comprised in a sample. Labeling typically is accomplished by binding a conjugate as specified herein below to the analyte, such that an indicator agent conjugated to a detection agent in the conjugate becomes non-covalently or covalently, in an embodiment non-covalently, associated with the analyte. Thus, the binding of the indicator agent to the analyte may in particular be indirect, i.e. via the detection agent. In an embodiment, as specified in more detail herein below, the analyte is comprised in a larger structure comprising a multitude of chemical compounds, in particular a cell, such as a eukaryotic cell, in which case the analyte may also be referred to as "marker" or "surface marker". In such case, as will be understood, analyte molecules present in the cell and/or on the cell surface will become labeled, causing the cell to become labeled as well. In an embodiment, in case a multitude of analytes is labeled in the same sample of cells, such analytes may be comprised on the same cell, e.g. in case of double-labeling of a cell, e.g. in CD3 and CD19 labeling, or may be present on different cell types, e.g. in case of labeling two different cell types, such as CD4+ T cells and CD8+ T cells. As will be understood by the skilled person, also mixed type labeling is possible, e.g. in case one analyte is present on a group of cells, and a second analyte is only present on a subgroup thereof; or in case a first marker is present on a first group of cells and a second marker is present on a second group of cells, but both the first and second analyte may be present on a further, third group of cells.

The term "analyte", as used herein, relates to a chemical molecule, in an embodiment, an organic molecule, binding to a detection agent with sufficient affinity to allow detection of an analyte/detection agent complex. In an embodiment, the dissociation constant (Kd) of the analyte/detection agent complex is at most 10⁻⁷ mol/L, in a further embodiment, at most 10⁻⁸ mol/l, in a further embodiment, at most 10⁻⁹ mol/L. In an embodiment, the analyte is a biological molecule, in a further embodiment, the analyte is a biological macromolecule. In a further embodiment, the analyte is a polypeptide. In case the analyte is comprised in a cell, such analyte may also be referred to as "marker" or "biomarker".

In an embodiment, in case the analyte is a polypeptide, the polypeptide is an antigen produced by an infectious agent, e.g., a virus, a bacterium, or a protozoan organism; or the analyte is an antibody produced by a subject against an antigen produced by an infectious agent, e.g., a virus, a bacterium, or a protozoan organism. The analyte may, however, also be a non-antibody polypeptide produced by a subject, in an embodiment being of diagnostic interest, e.g. folic acid binding protein or intrinsic factor. In a further embodiment, the analyte is a polypeptide comprised in a host cell, in particular exposed on the host cell surface. Thus, the analyte in an embodiment is a cell surface marker. Suitable cell surface markers are known in the art and are described herein elsewhere. In an embodiment, the analyte is a marker used in leukocyte and or thrombocyte and or erythrocyte differentiation, e.g. TBNK differentiation, used in CD4 immunophenotyping, e.g. in HIV infected subjects, or is a marker used in acute leukemia, in particular acute myeloid leukemia, differentiation. Thus, the analyte may be CD3, CD4, CD8, CD45, CD56, CD16, and/or CD19, as used e.g. in TBNK differentiation; and/or CD13, CD15, CD33, CD61, and/or CD9, as used e.g. in acute leukemia differentiation; and/or CD3, CD4, CD8, CD19, and/or CD20, as used e.g. in HIV leukocyte analysis. The analyte may, however, also be any other cell marker deemed suitable by the skilled person, including in particular CD3, CD4, CD8, CD10, CD19, and/or CD33, e.g. as BLAST panel; CD11b, CD34, and/or HLA-DR (=Human Leukocyte Antigen - DR isotype), e.g. in APL (Acute promyelocytic leukemia) screening; CD4, CD5, CD8, CD19, and/or CD20, e.g. in CD20 drug monitoring; CD41, CD61, CD62p, and/or PS (phosphatidylserine), e.g. in PLT-Function Testing and Therapy; and/or CD14, CD41, CD42a, CD61, CD62p, and/or PS, e.g. in Coronary Artery Disease /Atherosclerosis diagnostics.

In an embodiment, the analyte is a bacterial antigen, e.g. a Treponema pallidum antigen, a Borrelia antigen, a Brucella antigen, a Chlamydia antigen, a Mycoplasma antigen, a Listeria antigen, or an antigen derived from a unicellular organism such as protozoa, in an embodiment a Trypanosoma cruzi antigen. In an embodiment, the analyte is an antibody against a bacterial antigen, in a further embodiment, against a bacterial polypeptide. In an embodiment, the antibody is an anti-Treponema pallidum antibody, an anti-Borrelia antibody, an anti-Brucella antibody, an anti-Chlamydia antibody, an anti-Mycoplasma antibody, or an anti-Listeria antibody. In yet another embodiment, the analyte is an antibody against a unicellular organism, in an embodiment an anti-Trypanosoma cruzi antibody. In a further embodiment, the analyte is an anti-self antibody, i.e. an antibody recognizing an antigen produced by the subject itself, in an embodiment an anti-self antibody diagnostic and/or prognostic of disease, in an embodiment auto-immune disease. Diagnostic and prognostic anti-self antibodies are known in the art and include e.g. anti-nuclear antibodies indicative of systemic lupus erythematosus or polymyositis, anti-thyroid antibodies indicative of Hashimoto's thyroiditis or Grave's disease, anti-intrinsic factor antibodies indicative of anemia, and anti-tissue transglutaminase antibodies indicative of celiac disease or gluten sensitivity.

In an embodiment, the analyte is a viral antigen, e.g. an antigen of a virus, in an embodiment an antigen of a virus selected from the list consisting of Hepatitisvirus, Arbovirus, Adenovirus, Coxsackievirus, Echovirus, Influenzavirus, Parainfluenzavirus, Cytomegalovirus, Herpesvirus, Epstein-Barr virus, Mumpsvirus, Norovirus, Rotavirus, Rubellavirus, Measlesvirus, Human Immunodeficiency virus, Varicella-Zoster virus, Poliovirus, Coronavirus, and Enterovirus. In an embodiment, the analyte is an antibody against a viral antigen, in a further embodiment, against a viral polypeptide, or, in a further embodiment, against a viral capsid polypeptide, in an embodiment of a medically relevant virus, in an embodiment selected from the list as specified herein above. In an embodiment, the virus is Hepatitis A virus, Hepatitis B virus, Hepatitis C virus. In an embodiment, the viral capsid polypeptide is a Hepatitis virus capsid polypeptide, in an embodiment, a Hepatitis A (HA) virus capsid polypeptide. Accordingly, the analyte, in an embodiment, is an antibody against a Hepatitis virus capsid polypeptide, e.g., against a Hepatitis A virus capsid polypeptide.

In an embodiment, the analyte is a protozoan antigen, e.g. from Toxoplasma, in an embodiment T. gondii. In an embodiment, the analyte is an antibody against a protozoan antigen, in a further embodiment, against a protozoan polypeptide. In an embodiment, the analyte is an antibody against the T. gondii p30 polypeptide (Genbank Acc. No. S85174.1).

It is, however, also envisaged that the analyte is a low-molecular weight compound determinable by complex formation with a detection agent, in particular with an antibody or fragment thereof. In an embodiment, the analyte has a molecular mass of at least 100 (corresponding to 100 atomic mass units, and to 100 Da; 1 Da corresponding to 1.66×10-27 kg), in a further embodiment, at least 250, in a further embodiment, at least 500, or, in a further embodiment, at least 1000. In an embodiment, the analyte is thyroxin (T4), triiodothyronine (T3), folic acid, or vitamin B 12.

The term "sample", as used herein, relates to any sample known or suspected to comprise at least one analyte. The sample may be a liquid, semisolid, solid, or gaseous sample comprising or suspected to comprise at least one analyte, in an embodiment a liquid, semisolid, or solid sample. In an embodiment, the sample is a biological sample. Thus, the sample may e.g. be a sample comprising cells, in an embodiment host cells as specified herein below, so the sample may be a cell culture medium or harvested cultured cells. In an embodiment, the sample is a sample of a subject, such as a body fluid, a sample from a tissue or an organ, or a sample of wash/rinse fluid or a swab or smear obtained from an outer or inner body surface. In an embodiment, the sample is a blood, plasma, serum, urine, saliva, or lacrimal fluid sample. Samples can be obtained by use of brushes, (cotton) swabs, spatula, rinse/wash fluids, punch biopsy devices, puncture of a body cavity with a needle or a lancet, or by surgical instrumentation. However, samples obtained by other well-known techniques including, in an embodiment, scrapes, swabs or biopsies from the urogenital tract, perianal regions, anal canal, the oral cavity, the upper aerodigestive tract and the epidermis are also included as samples of the present invention. In an embodiment, the sample is a sample comprising cells of a subject; however, also cell-free fluids may be obtained from the aforesaid body fluids, tissues, or organs by lysing techniques such as homogenization and/or by separating techniques such as filtration or centrifugation. In an embodiment, samples are obtained from body fluids known or suspected to comprise one or more analyte(s). In an embodiment, the sample is an easily accessible sample, in an embodiment blood, plasma, serum, saliva, or the like, ore a sample derived therefrom, e.g. a sample of blood cells, in particular white blood cells, in an embodiment peripheral blood monocytes (PBMCs). It is to be understood that the sample may be further processed in order to carry out the method. Particularly, cells or specific cell types may be obtained or removed from the sample by methods and means known in the art. Moreover, at least one analyte may be extracted and/or purified from the sample by methods and means known in the art. As is understood by the skilled person, the specific method(s) of sample pretreatment will depend e.g. on sample type; e.g. gaseous samples may require extraction of analyte into a liquid medium before further use. Thus, the term sample also may relate to preparations comprising or suspected to comprise at least one analyte, diluted, enriched, purified and/or extracted from a sample.

The term "host cell" as used herein refers to a eukaryotic cell, in an embodiment known or suspected to comprise at least one analyte. In an embodiment, the host cell is a vertebrate cell, in a further embodiment a mammalian cell and, in a further embodiment, a human cell.

As used herein, the term "detection agent" relates to a compound comprising a structure binding, directly or indirectly, to the analyte, in an embodiment binding specifically to the analyte. In an embodiment, a detection agent and any additional detection agent, and optionally any further detection agent, are mutually non-identical.

The detection agent may be an indirect detection compound, i.e. a detection agent not contacting the analyte directly, but by means of a further compound which itself binds to the analyte; thus, the detection agent may e.g. be an anti-antibody antibody binding an anti-analyte antibody. In an embodiment, the detection agent is a direct detection agent, i.e. is an agent directly binding to the analyte. In an embodiment, the detection agent is an antibody or an analyte-binding fragment or derivative thereof, in an embodiment is an IgG or a fragment thereof. In an embodiment, the detection agent is a monoclonal antibody.

In an embodiment, the detection agent comprises at least one affinity domain, the term "affinity domain" relating to a substructure of the detection agent mediating affinity of the detection agent to an analyte. Thus, the affinity domain is the substructure of the detection agent comprising the atoms contacting the analyte in the detection agent/analyte complex. The affinity domain may comprise more than one affinity domains, e.g., in an embodiment two identical affinity domains, e.g. in case the affinity domain is an IgG, in a further embodiment ten identical affinity domains, e.g. in case the affinity domain is an IgM, in a further embodiment two different affinity domains in case the affinity domain is a diabody. In a further embodiment, the affinity domain may comprise a multitude, i.e. any number of at least 2, of identical or non-identical affinity domains in case the affinity domain is a polyepitope antigen. The detection agent may, e.g. in case it is a polypeptide, comprise further domains, e.g. structural domains not relevant for binding the analyte, such as e.g. the Fc domain of an antibody. In an embodiment, the affinity domain of a first detection agent is non-identical to the affinity domain of a second detection agent. Thus, in an embodiment, the first and the second detection agents may be different monoclonal antibodies, recognizing, in an embodiment, different analytes, or different epitopes of the same analyte.

In an embodiment, the detection agent further comprises one or more linkage group(s), the term "linkage group" relating to any chemical group enabling coupling of the detection agent to an indicator agent. Thus, the linkage group in an embodiment comprises at least one reactive or activatable chemical group enabling formation of a new chemical bond to a reaction partner. Appropriate chemical groups and related chemistries are known in the art; the skilled person selects appropriate groups and chemistries in particular depending on the chemical natures of the detection agent and the indicator agent. As is understood by the skilled person, the linkage group may be a chemical group already present in the detection agent, such as an amino or sulfhydryl group in a polypeptide. The linkage group may further comprise a linker, i.e. a chemical group extended in one direction, which in an embodiment increases distance and/or flexibility between the detection agent and its coupling partner, in an embodiment the indicator agent.

In an embodiment the coupling between the detection agent and the indicator agent is non-covalent; thus, the detection agent in an embodiment comprises one partner of an affinity pair. The term "affinity pair" is understood by the skilled person. In an embodiment, the term relates to a pair of compounds, in an embodiment polypeptides, forming a complex in an aqueous solution, in an embodiment under standard conditions. In an embodiment, the complex between the components of the affinity pair has a dissociation constant of at most 10⁻⁹ mol/l, in an embodiment at most 10⁻¹⁰ mol/l, in a further embodiment at most 10⁻¹¹ mol/l, in a further embodiment at most 10⁻¹² mol/l, in a further embodiment at most 10⁻¹³ mol/l, in a further embodiment at most 10⁻¹⁴ mol/l. In an embodiment, the affinity pair comprises, in a further embodiment consists of, a first affinity partner and a second affinity partner as specified elsewhere herein. Affinity pairs are, in principle, known in the art, and include in particular biotin-streptavidin affinity pairs and affinity pairs derived therefrom, such as strep-tag and streptactin derivatives; digoxigenin and anti-digoxigenin (i.e. an antibody against digoxigenin) pairs, colicin-immunity pairs, and the like. However, in principle, any pair of compounds with sufficient affinity and specificity may be used, including e.g. myc-tag-antibody and flag-tag-antibody pairs.

As referred to herein, the terms "first partner of an affinity pair" and "first affinity partner", "second partner of an affinity pair" and "second affinity partner", "one affinity partner" and "other affinity partner", are used herein solely to differentiate between the two partners of the affinity pair; thus, the expression "first partner" does not relate to any timely or spatial relation to other compounds, but is used only for the purpose of differentiation from the "second partner" of an affinity pair. The first affinity partner and the second affinity partner, and the one and the other affinity partner, together constitute the affinity pair as specified herein above, respectively. The aforesaid applies mutatis mutandis to the terms "detection agent" and "additional detection agent", to the terms "indicator agent" and "additional indicator agent", and to the terms "first reaction vessel", "second reaction vessel", and "third reaction vessel", and similar expressions.

The term "indicator agent", as used herein, relates to a compound adapted for making the presence of a molecule or complex comprising said indicator agent detectable. Typically, the indicator has a detectable property, in an embodiment a detectable physical, and/or chemical property. In an embodiment, an indicator agent and any optional further indicator agent(s) used in the method, are mutually non-identical.

As used herein, the term "physical property" relates to any property which is detectable by physical means, and includes in particular optical properties, electrochemical properties, and emission of radiation. The term "optical property", as used herein, relates to any property which can be detected by an optical instrument. Specifically, the optically determinable property may be or may comprise at least one property selected from the group consisting of a reflection property, a transmission property, an emission property, a scattering property, a fluorescence property, a phosphorescence property, a diffraction property, and a polarization property. Thus, optical properties envisaged by the present invention e.g. are color, fluorescence, luminescence, or refraction. In an embodiment, an optically determinable property as referred to herein refers to a property of a chemical compound which can be optically detected such as light absorption, light emission, light remission, or properties associated therewith. It will be understood that detecting an optically determinable property as used herein encompasses the detection of the presence of a property which was not detectable before, the detection of the absence of a property which has been detected before, and the detection of quantitative changes of a property, i.e., the detection of the change of the signal strength which correlates to the extent of the change of the at least one optical property. In accordance, the indicator agent is or comprises a fluorophore, in an embodiment selected from the list consisting of DAPI, fluorescein, rhodamine, eosin, CF4055^{™}, Brilliant Violet 510TM, CF488A^{™}, APC-Vio770^{™}, or StarBright Violet 610^{™}. It is understood that the term "optically determinable property", in an embodiment, also relates to electrochemiluminescence, which is also known as electrogenerated chemiluminescence.

The term "chemical property" as used herein, relates to all features detectable by at least one chemical reaction, e.g. the activity of producing a chemical compound, in an embodiment when provided with a co-substrate and/or a catalyst; as referred to herein, a chemical property may be the activity of being a catalyst, e.g. an enzymatic property. The term "enzymatic property", as used herein, relates to a property of an indicator agent of producing a detectable product from a substrate by means of biological catalysis. Accordingly, an enzymatic property is typically conferred by the presence of a polypeptide having said enzymatic property in said indicator agent. Typically, the enzymatic property selected from the group consisting of phosphatase activity (e.g. in alkaline phosphatase), peroxidase activity (e.g. in horseradish peroxidase), and glycosidase activity (e.g. in beta-galactosidase). Typical substrates for enzymatic activities are well-known in the art. Typically, said enzymatic activity produces a product having a determinable optical property as specified herein above, or/and said enzymatic activity produces a product being determinable by an electrical instrument.

The term "conjugating" is understood by the skilled person. As used herein, the term in an embodiment relates to forming and/or providing a, covalent or non-covalent, bond between chemical molecules to provide a conjugate. Said bond in an embodiment is stable as to allow the conjugate to be detected, in an embodiment by the detectable property of the indicator agent. Thus, the conjugate in an embodiment has a dissociation constant lower than the dissociation constant for the analyte-binding agent complex, in an embodiment in the range described for affinity pairs, both as specified herein above. In a further embodiment, conjugating is forming at least one covalent bond between chemical molecules to provide a conjugate. In an embodiment, conjugating is forming and/or providing a conjugate between a detection agent and an indicator agent.

In accordance with the above, the term "conjugation product", which may also be referred to as "conjugate", relates to any chemical molecule or complex of chemical molecules comprising at least one binding agent and at least one indicator agent, in an embodiment formed by conjugation, in an embodiment all as specified herein above. Thus, the conjugate may in particular be a dye-labeled binding agent, such as a dye-labeled antibody or fragment thereof. The conjugate may be purified, e.g. to remove non-conjugated binding agent and/or indicator agent; the conjugate may also be pretreated before use, e.g. to protect and/or neutralize any non-reacted linkage groups or other reactive groups before use; in an embodiment, however, the conjugate is used in the methods of the present invention as the preparation which is obtained in the conjugation step, i.e. in an embodiment without any intervening purification and/or pretreatment steps.

The term "contacting" as used in the context of the methods specified herein is understood by the skilled person. In an embodiment, the term relates to bringing at least one compound, in particular a conjugate, in physical contact with a sample and/or with a further compound and thereby, e.g. allowing the sample and the compound to interact.

As the skilled person understands in view of the description herein, the term "directly" has a timely and a spatial/order-related component. Thus, the expression "performing step (b) directly after completion of step (a)", in an embodiment, relates (i) to performing step (b) timely directly after completion of step (a), (ii) to performing step (b) in the order of steps of the method directly after completion of step (a), (iii) to performing step (b) on the same device on which step (a) is performed, or any combination of (i) to (iii), in particular all of the aforesaid. As used herein, the term "timely directly" is used in a broad sense and relates to a time frame of at most 1 day, in an embodiment at most 12h, in a further embodiment at most 6h, in a further embodiment at most 3h, in a further embodiment at most 1h, in a further embodiment at most 0.5h, in a further embodiment at most 0.25h, after completion of step (a). In a further embodiment, the term timely directly relates to a time frame as short as technically possible. As will be understood by the skilled person, a time frame as short as technically possible, in an embodiment, is the period of time an operator or device requires for adding a conjugate to a sample after completion of step (a); for the avoidance of doubt, as referred to herein, the term "after completion of step (a)" relates to a time frame starting when it is detected or decided that step (a) was completed. The term "directly in the order of steps" is understood by the skilled person. In an embodiment, the term relates to the fact that the next step after completion of step (a) is step (b), i.e. in an embodiment without any step intervening steps (a) and (b). As referred to herein, relevant steps in determining the order of steps are those relating to handling of detection agent, indicator agent, and/or sample. Thus, in the method as referred to herein, any auxiliary or non-related step may also be performed between steps (a) and (b) for the order to be directly; such auxiliary or non-related steps may e.g. be discarding surplus reagent(s) to a waste or transferring a sample to a reaction vessel, e.g. a second reaction vessel as specified herein above. In accordance with the above, in an embodiment step (a) is performed in a first reaction vessel and performing directly comprises contacting the conjugate from step (a) with the sample in a second reaction vessel, wherein the conjugate from step (a) is not contacted to any further vessel before step (b). In such case, the conjugation product of step (a) may be transferred from the first reaction vessel into the second reaction vessel, in an embodiment wherein the conjugate exclusively contacts the first reaction vessel, a means of pipetting, and the second reaction vessel and the sample during steps (a) and (b). In an embodiment, performing step (b) directly after completion of step (a) comprises contacting the conjugate with the sample within one of the time frames indicated herein above and with step (b) being performed with no intervening steps after completion of step (a). Thus, in particular, the conjugate in an embodiment is not stored for extended periods of time, in particular not longer than 1 day. In a further embodiment, the conjugate is prepared specifically for a detection reaction or a series of detection reactions in which at least the analyte detected by the conjugate is common to all detection reactions. The term "on the same device on which step (a) is performed" is understood by the skilled person and in an embodiment relates to the fact that both steps (a) and (b) are performed on the same device. In an embodiment, said device is a labeling device as specified herein below.

A method of the present invention comprising providing a detection agent binding to the analyte and conjugating said detection agent with an indicator agent to produce a conjugation product, followed by direct use of the conjugation product as specified herein may also be referred to as "on-board conjugation" herein.

The term "subject", as used herein, relates to a vertebrate, in an embodiment mammalian subject. In an embodiment, the subject is a farm animal, a companion animal, or an experimental animal, e.g. cattle, sheep, goat, horse, cat, dog, guinea pig, mouse, or rat. In an embodiment, the subject is a human. In a further embodiment, the subject is a human suspected to comprise an analyte as specified herein.

The method for labeling an analyte in a sample described herein comprises step (a) providing a detection agent binding to the analyte and conjugating said detection agent with an indicator agent to produce a conjugation product.

The term "providing a detection agent" is understood by the skilled person as relating to making available for further use. In an embodiment, providing is making physically available a detection agent. The detection agent, in an embodiment is a detection agent known to bind to the analyte; thus, providing a detection agent in an embodiment does not comprise identifying a detection agent, e.g. in a screening method. Typically, providing a detection agent may in particular be ordering and receiving a commercially available detection agent or producing a detection agent known to bind to an analyte.

Conjugating a detection agent with an indicator agent has been described herein above. The product of said conjugating, i.e. the conjugate, may be used in step (b) as it is obtained in the conjugating step, or may be purified and/or pretreated as described herein above. In an embodiment, the conjugate is used in step (b) as it is obtained in the conjugating step.

The method for labeling an analyte in a sample described herein further comprises step (b) contacting a sample with said conjugation product of step (a). The term contacting has been specified herein above. In an embodiment, the term relates to bringing a conjugate and a sample in physical contact. Step (b) may comprise bringing the aforesaid compounds into contact with further compounds and elements, depending on the assay format chosen; e.g. in assays relying on removal of non-complexed sample constituents via binding of complexes to a solid surface, the admixture may be further contacted with a solid surface. Also, in assays in which the analyte is comprised in or on the surface of a cell, step (b) may further comprise contacting the cells with a solid surface, e.g. of a bead, enabling separation of cells from sample constituents and/or reagents of step(s) (a) and/or (b). Also, step (b) may comprise contacting the sample to further reagents like in particular buffers, osmotic stabilizers, salts, nutrients, and the like. As described herein, step (b) is performed directly after completion of step (a).

In an embodiment, the method for labeling an analyte in a sample described herein further comprises an optional additional step (a1) of providing an additional detection agent binding to an additional analyte, an additional step of conjugating said additional detection agent with an additional indicator agent to produce an additional conjugation product, wherein step (b) comprises contacting said sample with the conjugation product of step (a), and with said additional conjugation product of step (a1), wherein step (b) is performed directly after completion of at least one of said providing and conjugating steps. Also, step (a1) typically is performed spatially separate from step (a), in an embodiment is performed in a separate reaction vessel. Thus, in an embodiment, conjugating step (a) is performed in a first reaction vessel and step (a1) is performed in a third reaction vessel, wherein performing directly comprises contacting the conjugates from steps (a) and (a1) with the sample in the second reaction vessel, wherein in an embodiment the conjugate from step (a) and/or the conjugate from step (a1) is not contacted to any further vessel before step (c). Thus, in an embodiment, the detection agent, the at least one additional detection agent, and the sample in an embodiment are contacted concomitantly in step (b). It is, however, also envisaged that the sample is contacted with the detection agent and the additional detection agent successively, in an embodiment in any order deemed suitable by the skilled person. In a further embodiment, the method comprises at least one further step (a2) of providing a further detection agent binding to a further analyte, a further step of conjugating said further detection agent with a further indicator agent to produce a further conjugation product, wherein step (b) comprises contacting said sample with the conjugation product of step (a), with the additional conjugation product of step (a1), and with the at least one further conjugation product of step (a2), wherein step (b) in an embodiment is performed directly after completion of at least one of said providing and conjugating steps. Any additional conjugate may, in principle, be provided also in a pre-manufactured form, e.g. as commercially available, such that only the conjugate of step (a) is contacted directly with the sample as the term is used herein. In an embodiment, however, at least two, in an embodiment all, conjugates contacted with the sample are provided and conjugated as specified for step (a) and, in an embodiment, contacting of at least two, in an embodiment all, of said conjugates is performed directly after completion of conjugation as specified herein above. Thus, in an embodiment, in the method for labeling an analyte in a sample at least two, in an embodiment all, conjugates are produced directly before the contacting step. In accordance, in an embodiment, the method relates to contacting the sample with a multitude of conjugates, in an embodiment to label a multitude of analytes. The additional detection agent typically is non-identical to any further detection agent used in the method; also the additional indicator agent typically is non-identical to any further indicator agent used in the method.

In an embodiment, the indicator agent, an additional indicator agent, and optionally any further indicator agent, are selected such that interference between said indicator agents is reduced, wherein said interference in an embodiment is selected from the list consisting of fluorescence spillover, overlap of absorption spectra, and cross-excitation. Said reduction of interference in an embodiment is a reduction of said interference by at least 25%, in an embodiment at least 50%, in a further embodiment at least 75%; reduction may, however, also be complete reduction, i.e. elimination of said interference. As the skilled person understands, it may be sufficient if at least one of the aforesaid interferences is reduced as specified.

Unless specifically indicated otherwise herein, the compounds specified, in particular the analytes, detection agents, indicator agents, and conjugates thereof, may be comprised in larger structures, e.g. may be covalently or non-covalently linked to further molecules or substructures. In particular, detection agents as specified may be comprised in fusion polypeptides comprising further peptides, which may serve e.g. as a tag for purification and/or detection, as a linker, or may be a partner of an affinity pair as specified elsewhere herein. The term "detectable tag" refers to a stretch of amino acids which are added to or introduced into the fusion polypeptide; in an embodiment, the tag is added C- or N- terminally to the fusion polypeptide. Said stretch of amino acids in an embodiment allows for detection of the polypeptide by an antibody which specifically recognizes the tag; or it in an embodiment allows for forming a functional conformation, such as a chelator; or it in an embodiment allows for visualization, e.g. in the case of fluorescent tags. Preferred detectable tags are the Myc-tag, FLAG-tag, 6-His-tag, HA-tag, GST-tag or a fluorescent protein tag, e.g. a GFP-tag. These tags are all well known in the art.

Advantageously, it was found in the work underlying the present invention that conjugates between detection agents and indicator agents can be synthesized on board, e.g. on a labeling device as specified herein below. Such proceeding enables tailored selection of indicator agents to reduce interferences between indicator agents, but also selection of indicator agents according to availability, e.g. providing for improved and more efficient reagent management.. Thus, the latter option enables to use up indicator reagents which would have to be discarded if provided as pre-manufactured conjugates which may not be used up by the use-by date. Regarding the former, the method also enables selection of favorable combinations of indicator agents, making separation of detection channels between indicators agents easier.

The definitions made above apply mutatis mutandis to the following. Additional definitions and explanations made further below also apply for all embodiments described in this specification mutatis mutandis.

The present invention further relates to a labeling device comprising
(A) a first container comprising a detection agent, operably connected to a reaction unit;
(B) a second container comprising an indicator agent, operably connected to the reaction unit, and
(C) a labeling unit;
wherein the reaction unit is configured for conjugating said detection agent with the indicator agent to produce a conjugation product, and wherein said labeling unit is configured to contact a sample with the conjugation product.

The term "labeling device", as used herein, relates to a system of means comprising at least the means described operatively linked to each other as to allow the conjugating and labeling. Typical means for conjugating a detection agent and an indicator agent are known in the art and have been described herein above in the context of the methods of the invention. In an embodiment, means for conjugating include a reaction vessel, e.g. at least a first and a second reaction vessel as specified herein above. It is, however, also envisaged that the sample is added to the conjugate directly after conjugation into the vessel in which the conjugation as performed; thus, the device may also comprise only one reaction vessel. In a further embodiment, the device comprises a multitude of reactions vessels, which are in an embodiment disposable; thus, the reaction vessels may e.g. be multi-well plates. In an embodiment, the labeling device is adapted to perform a method comprising the method for labeling an analyte in a sample as specified herein above. Thus, in an embodiment, the labeling device further comprises a processor and, in an embodiment tangibly embedded, instructions which, when performed on the processor, cause the labeling device to perform the steps of the method for labeling an analyte in a sample as specified herein elsewhere.

The labeling device comprises a reaction unit. As used herein, the term "reaction unit" relates to a unit of the device comprising means for conjugating said detection agent with the indicator agent to produce a first conjugation product; in an embodiment, the conjugating is performed in a first reaction vessel and the detection reagent and the indicator agent are transferred into said reaction vessel via a pipetting mechanism. As the skilled person understands in view of the description herein above, the reaction unit in an embodiment comprises means for performing more than one conjugation, in an embodiment a plurality of conjugations, in an embodiment at least two, in a further embodiment at least three, in a further embodiment at least four, conjugations. Said conjugations are, in an embodiment, performed simultaneously or with overlapping time frames; thus the reaction unit in an embodiment comprises a multitude of reaction vessels and corresponding pipetting mechanism.

The labeling device comprises a labeling unit, the term "labeling unit", as referred to herein, relating to any unit adapted for contacting a conjugate as specified herein above with a sample. Thus, the labeling unit in an embodiment comprises a reaction vessel, e.g. a second reaction vessel as described herein above, and a pipetting mechanism allowing the transfer of a sample and/or at least one conjugate into said reaction vessel. Depending on the type of labeling performed, the labeling unit may comprise further elements, such as a magnet for performing magnetic bead assisted cell sorting or for immobilizing a complex comprising the detection agent, the indicator agent, and the analyte. However, as the term is referred to herein, the term labeling unit relates to any unit adapted for contacting a conjugate as specified herein above with a sample, in an embodiment allowing formation of a complex comprising the analyte, the detection agent, and the indicator agent. Removal of surplus reagents and/or non-analyte sample constituents (matrix) may be optional, depending on the further use of the labeled analyte intended.

In an embodiment, the labeling unit is operably connected to an analysis unit comprising a detector, wherein the detector is adapted for detecting the conjugation product, in an embodiment by means of the detectable property of the indicator agent. Appropriate means are as such well-known in the art and include in particular photo-optical units such as a photometer, a fluorimeter, a digital imaging unit, a microscope, such as a digital microscope, an in-stream imaging unit of a fluorescence activated cell sorting (FACS) device, and similar units. Digital microscopes and means for producing slides for automatic evaluation are described e.g. in US 9,017,610 B2. The detector may further comprise a means for separating complexes comprising the indicator agent from other compounds, in particular matrix constituents, unlabeled cells, and the like. Thus, the detector may e.g. be a cell sorting unit, in an embodiment a FACS unit.

In an embodiment, the labeling device further comprises a selector unit. As referred to herein, the term "selector unit" relates to a unit adapted for selecting one or more combinations of detection agents and indicator agents for conjugation. Thus, selector unit in an embodiment comprises tangibly embedded an algorithm for selecting an indicator agent for conjugation with a detection agent, in an embodiment for selecting at least a first indicator agent for conjugation with a first detection agent and a second indicator agent for conjugation with a second detection agent. In an embodiment, the selection is such that interferences as specified herein above are reduced or avoided entirely; thus, the selector unit may further comprise, preferably tangibly embedded, a database comprising data on indicator compounds allocated to data on interfering indicator compounds and/or interferences.

The labeling device may also comprise an evaluation unit, wherein the term "evaluation unit" relates to a unit adapted to process and optionally analyze data produced by the analysis unit. Thus, evaluation unit of the device in an embodiment comprises a memory unit comprising tangibly embedded at least one algorithm for evaluating data received by the evaluation unit from the analysis unit. Appropriate evaluation units are known in the art, e.g. as units of ELISA meters, FACS devices, automated microscope slide analyzers, and the like.

Further units of the device which may optionally be present may be related to the requirements of the conjugating and labeling reactions intended. I.e., e.g. in the case of conjugating via an affinity pair, it may be sufficient to mix the detection agent bound to a first partner of an affinity pair with an indicator agent bound to a corresponding second partner of an affinity pair, in an embodiment under standard conditions. On the other hand, coupling via a chemical reaction forming a covalent bond between the detection agent and the indicator agent may require deviation from standard conditions, e.g. increased and/or decreased temperature, increased and/or decreased pH, intensive stirring, or the like. Similarly, the labeling may require modifying conditions and/or additional reagents. Thus, in an embodiment, the reaction unit and/or the labeling unit further comprise(s), independently selected, a heating unit, a cooling unit, one or more co-reactant(s) and/or catalyst(s), a buffer, a dilution agent, and/or a stopping agent, a stirrer, a shaker, and/or a pipetting unit.

Further units of the device which may optionally be present may be related to further functions which may be performed by the device; such units may in particular be a control unit controlling further functions and/or units of the device, such as a washing unit for separating complexes comprising the analyte, the detection agent and the indicator agent from sample constituents, from non-complexed detection agent, and/or from non-complexed indicator agent; an output unit, such as a monitor, a printer, a network connection, and/or other means of conveying information to a user and/or one or more connected devices; an input unit; and/or a data storage unit, such as a memory, or a mass storage device and/or a data interface to an external data storage device.

How to link the means of the labeling device in an operating manner will depend on the type of means included into the labeling device. In an embodiment, the means are comprised by a single device. However, it is also contemplated that the means of the current invention, in an embodiment, may appear as separate devices and are, in a further embodiment, packaged together as a kit. The person skilled in the art will realize how to link the means without further ado. Preferred devices are those which can be applied without the particular knowledge of a specialized technician. In an embodiment, the device is adapted to include an additional feature as described herein.

The present invention also relates to a system comprising, operably connected,
(i) a labeling device according to the present invention; and
(ii) a detection device and/or a selector device.

The labeling device has been described herein above. The term "detection device", as used herein, relates to any device comprising a detector as specified herein above in a discrete device, and may in particular be a FACS device or a digital imaging device. Similarly, the term "selector device", as used herein, relates to any device comprising a selector unit as specified herein above in a discrete device. As the skilled person understands, a labeling device which is part of a system as specified herein in an embodiment does not comprise a detector and/or does not comprise a selector unit.

In the system, the devices are operably connected. In view of the description provided herein, the skilled person knows how to operably connect the devices of the system. In an embodiment, the detection device is connected to said labeling device by a fluid connection, wherein said fluid connection may be any means adapted for transferring fluid from one device to another device, such as one or more tubing with an optional pump, or a transport mechanism for transporting one or more reaction vessels, such as a transport belt or a robotic arm. The selector device may provide data on one or more combinations of detection agent(s) and indicator agent(s) to the labeling device; thus, the operable connection between the labeling device and the selector device may be a data transfer connection. In accordance, the selector device may be any device comprising a means adapted for selecting one or more combinations of detection agents and indicator agents for conjugation. Thus, the selector device may in principle be any device comprising a processor and at least one appropriate algorithm and optionally a database, both as specified herein above. In accordance, the device may e.g. be an appropriately adapted mobile device such as a mobile phone or a tablet computer, or a desktop computer, connected to the labeling device via a data transfer connection.

The present invention also relates to an, in an embodiment computer-implemented, method of selecting at least two indicator agents for labeling two analytes with two detection agents, comprising
(I) obtaining data on available indicator agents
(II) obtaining data on the analytes; and
(III) selecting at least two indicator agents based on the data obtained in steps (I) and (II), wherein said selection fulfills at least one condition selected from the list consisting of
   - reduction or prevention of fluorescence spillover,
   - reduction or prevention of overlap of absorption spectra,
   - reduction or prevention of cross-excitation.

The method of selecting of the present invention is an in vitro method, in an embodiment an in silico method. Moreover, the method may comprise steps in addition to those explicitly mentioned above. Moreover, one or more of said steps may be assisted or performed by automated equipment. In particular, the method may be implemented in a labeling device or a selector device as specified elsewhere herein.

The term "data on available indicator reagents" is understood by the skilled person. In an embodiment, the term relates to data relating to indicator agents available for conjugation with a detection agent as specified herein above. Such data may relate to indicator agents in principle available, e.g. commercially; in an embodiment, the term relates to indicator agents accessible for conjugation at the time the method of selecting is performed. Thus, the data on available indicator agents may in particular relate to indicator agents available in a particular laboratory, or on a particular device. Thus, in case the indicator compound has an optically detectable property, the data provided for the indicator agent may e.g. be available volume, use-by date, indicator identifier, absorption maximum or maxima, absorption range, absorption spectrum, emission maximum or maxima, and/or emission range.

The term "data on the analyte" is also understood by the skilled person. In an embodiment, said data are data relevant for the selection of indicator agent(s), such as e.g. expected concentration range of the analyte(s), photo-optical properties of the analyte(s), possible reactivity with indicator reagents, and the like. In case more than one indicator agent is planned to be used, such data may also comprise data on a number of identical and/or non-identical markers in or on said analyte, e.g. a cell.

The term "fluorescence spillover" is known to the skilled person to relate to emission of a first indicator agent in a wavelength range used for detection of a second indicator agent; The term "overlap of absorption spectra" is known to the skilled person to relate to the fact that a first and a second indicator agent may have absorption spectra which overlap, such that excitation in the overlapping range may lead to reduced excitation of one or both of said indicator agent(s). The term "cross-excitation" is known to relate to the fact that an emission by a first indicator agent may lie in the absorption range of a second indicator reagent, potentially causing non-intended excitation of the second indicator agent.

As referred to herein, measures for avoiding the aforesaid interferences relate to selection of indicator agent and, thus, in an embodiment, do not relate to device-side compensation methods such as use of filters, unmixing, and the like. Thus, the aforesaid conditions are fulfilled by a suitable selection of indicator agents, in particular such that emission ranges do not overlap between at least two, in an embodiment all, indicator agents, absorption ranges do not overlap between at least two, in an embodiment all, indicator agents, and/or an emission range of a first indicator does not overlap with an excitation range of a second indicator agent.

Also, the present invention relates to a use of on-board conjugation for producing at least two detection agents comprising non-identical indicator agents on a device comprising an analysis unit and a detector.

The invention further discloses and proposes a computer program including computer-executable instructions for performing the method according to the present invention in one or more of the embodiments enclosed herein when the program is executed on a computer or computer network. Specifically, the computer program may be stored on a computer-readable data carrier. Thus, specifically, one, more than one or even all of method steps a) to d) as indicated above may be performed by using a computer or a computer network, in an embodiment by using a computer program.

The invention further discloses and proposes a computer program product having program code means, in order to perform the method according to the present invention in one or more of the embodiments enclosed herein when the program is executed on a computer or computer network. Specifically, the program code means may be stored on a computer-readable data carrier.

Further, the invention discloses and proposes a data carrier having a data structure stored thereon, which, after loading into a computer or computer network, such as into a working memory or main memory of the computer or computer network, may execute the method according to one or more of the embodiments disclosed herein.

The invention further proposes and discloses a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier. Specifically, the computer program product may be distributed over a data network.

Finally, the invention proposes and discloses a modulated data signal which contains instructions readable by a computer system or computer network, for performing the method according to one or more of the embodiments disclosed herein.

In an embodiment, referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, the present invention further discloses:
- A computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

Summarizing the findings of the present invention, the following embodiments are particularly envisaged:
Embodiment 1: A method for labeling an analyte in a sample, said method comprising
   (a) providing a detection agent binding to the analyte and conjugating said detection agent with an indicator agent to produce a conjugation product;
   (b) contacting said sample with said conjugation product of step (a);
   (c) wherein said step (b) is performed directly after completion of step (a).
Embodiment 2: The method of embodiment 1, wherein performing directly comprises performing step (b) at most 1 day, in an embodiment at most 12h, in a further embodiment at most 6h, in a further embodiment at most 3h, in a further embodiment at most 1h, after completion of step (a).
Embodiment 3: The method of embodiment 1 or 2, wherein the conjugating step (a) is performed in a first reaction vessel and wherein performing directly comprises contacting the conjugate from step (a) with the sample in a second reaction vessel, wherein the conjugate from step (a) is not contacted to any further vessel before step (b).
Embodiment 4: The method of any one of embodiments 1 to 3, wherein said detection agent is an antibody or fragment thereof.
Embodiment 5: The method of any one of embodiments 1 to 4, wherein the coupling between the detection agent and the indicator agent is non-covalent, in an embodiment via an affinity pair, or is covalent, in an embodiment via an amido or a disulfide group.
Embodiment 6: The method of any one of embodiments 1 to 5, wherein the conjugation is performed in a first reaction vessel in step (a) and the conjugation product is transferred from said first reaction vessel into a second reaction vessel.
Embodiment 7: The method of any one of embodiments 1 to 6, wherein said method comprises an additional step (a1) of providing an additional detection agent binding to an additional analyte, an additional step of conjugating said additional detection agent with an additional indicator agent to produce an additional conjugation product, wherein step (b) comprises contacting said sample with the conjugation product of step (a), and with said additional conjugation product of step (a1), and wherein step (b) is performed directly after completion of at least one of said providing and conjugating steps.
Embodiment 8: The method of step 7, wherein the conjugating step (a) is performed in a first reaction vessel and step (a1) is performed in a third reaction vessel, wherein performing directly comprises contacting the conjugates from steps (a) and (a1) with the sample in the second reaction vessel, wherein the conjugate from step (a) and/or the conjugate from step (a1) is not contacted to any further vessel before step (c).
Embodiment 9: The method of embodiment 7 or 8, further comprising at least one further step (a2) of providing a further detection agent binding to a further analyte, a further step of conjugating said further detection agent with a further indicator agent to produce a further conjugation product, wherein step (b) comprises contacting said sample with the conjugation product of step (a), with the additional conjugation product of step (a1), and with the at least one further conjugation product of step (a2), and wherein step (b) is performed directly after completion of at least one of said providing and conjugating steps.
Embodiment 10: The method of any one of embodiments 1 to 9, wherein said analyte is a cell surface marker.
Embodiment 11: The method of any one of embodiments 7 to 10, wherein said additional analyte any optionally any further analyte(s), is a further cell surface marker.
Embodiment 12: The method of any one of embodiments 7 to 11, wherein the detection agent and the additional detection agent, and optionally any further detection agent, are mutually non-identical.
Embodiment 13: The method of any one of embodiments 7 to 12, wherein the indicator agent and the additional indicator agent, and optionally any further indicator agent, are mutually non-identical.
Embodiment 14: The method of any one of embodiments 7 to 13, wherein said indicator agent, said additional indicator agent, and optionally any further indicator agent(s), are non-identical dyes, in an embodiment non-identical fluorophores.
Embodiment 15: The method of any one of embodiments 7 to 14, wherein said method comprises selecting the indicator agent and additional indicator agent, and optionally any further indicator agent, such that such that interference between said indicator agents is reduced.
Embodiment 16: The method of any one of embodiments 7 to 15, wherein said interference is selected from the list consisting of fluorescence spillover, overlap of absorption spectra, cross-excitation.
Embodiment 17: The method of any one of embodiments 1 to 16, wherein said sample is a biological sample.
Embodiment 18: The method of any one of embodiments 1 to 17, wherein said sample comprises cells, in an embodiment human cells.
Embodiment 19: The method of any one of embodiments 1 to 18, wherein said method is implemented on a device according to any one of embodiments 24 to 35.
Embodiment 20: The method of any one of embodiments 1 to 19, wherein said conjugating comprises formation of a non-covalent association product of an affinity pair, or comprises formation of at least one covalent bond.
Embodiment 21: A method for labeling at least two analytes in a sample, said method comprising
   (aa) providing a first detection agent binding to a first analyte; and conjugating said first detection agent with a first indicator agent to produce a first conjugation product;
   (bb) providing a second detection agent binding to a second analyte; and conjugating said second detection agent with a second indicator agent to produce a second conjugation product;
   (cc) contacting said sample with said first conjugation product of step (aa) and with said second conjugation product of step (bb);
   wherein said step (cc) is performed directly after completion of step (bb).
Embodiment 22: A method for labeling at least two analytes in a sample, said method comprising
   (aaa) providing a first detection agent binding to a first analyte and conjugating said first detection agent with a first indicator agent to produce a first conjugation product; and/or providing a predetermined conjugation product being a predetermined detection agent conjugated to a predetermined indicator agent;
   (bbb) providing a second detection agent binding to a second analyte and conjugating said second detection agent with a second indicator agent to produce a second conjugation product;
   (ccc) contacting said sample with said first conjugation product and/or said predetermined conjugation product of step (aaa) and with said second conjugation product of step (bbb);
   wherein said step (ccc) is performed directly after completion of step (bbb).
Embodiment 23: A method of embodiment 21 or 22, further having a feature of any one of embodiments 1 to 20.
Embodiment 24: A labeling device comprising
   (A) a first container comprising a detection agent, operably connected to a reaction unit;
   (B) a second container comprising an indicator agent, operably connected to the reaction unit, and
   (C) a labeling unit;
   wherein the reaction unit is configured for conjugating said detection agent with the indicator agent to produce a conjugation product, and wherein said labeling unit is configured to contact a sample with the conjugation product.
Embodiment 25: The labeling device of embodiment 24, wherein said reaction unit comprises a first reaction vessel and optionally a second vessel..
Embodiment 26: The labeling device of embodiment 24 or 25, wherein said reaction unit further comprises, independently selected, a heating unit, a cooling unit, one or more co-reactant(s) and/or catalyst(s), a buffer, a dilution agent, and/or a stopping agent, a stirrer, a shaker, and/or a pipetting unit.
Embodiment 27: The labeling device of any one of embodiments 24 to 26, wherein said labelling unit further comprises a heating unit, a cooling unit, one or more co-reactant(s) and/or catalyst(s), a buffer, a dilution agent, and/or a stopping agent, a stirrer, a shaker , a pipetting unit.
Embodiment 28: The labeling device of any one of embodiments 24 to 27, wherein said labeling unit is operably connected to an analysis unit comprising a detector.
Embodiment 29: The labeling device of embodiment 28, wherein said detector is adapted for detecting the conjugation product.
Embodiment 30: The labeling device of any one of embodiments 24 to 29, wherein said detector comprises a FACS unit or a digital imaging unit.
Embodiment 31: The labeling device of any one of embodiments 24 to 30, further comprising a selector unit.
Embodiment 32: The labeling device of any one of embodiments 24 to 24, wherein said selector unit comprises tangibly embedded an algorithm for selecting an indicator agent for conjugation with a detection agent, in an embodiment for selecting at least a first indicator agent for conjugation with a first detection agent and a second indicator agent for conjugation with a second detection agent.
Embodiment 33: The labeling device of any one of embodiments 24 to 32, wherein said selector unit further comprises, in an embodiment tangibly embedded, a database comprising data on indicator compounds allocated to data on interfering indicator compounds and/or interferences.
Embodiment 34: The labeling device of any one of embodiments 24 to 33, adapted to perform the method of any one of embodiments 1 to 23.
Embodiment 35: The labeling device of any one of embodiments 24 to 34, further comprising a processor and, in an embodiment tangibly embedded, instructions which, when performed on the processor, cause the device to perform the method of any one of embodiments 1 to 23.
Embodiment 36: A system comprising, operably connected,
   (i) a labeling device according to any one of embodiments 24 to 35; and
   (ii) a detection device and/or a selector device.
Embodiment 37: The system of embodiment 36, wherein said detection device comprises a detector as specified in any one of embodiments 28 to 30.
Embodiment 38: The system of embodiment 36 or 37, wherein said detection device is a FACS device or a digital imaging device.
Embodiment 39: The system of any one of embodiments 36 to 38, wherein said detection device is connected to said labeling device by a fluid connection.
Embodiment 40: The system of any one of embodiments 36 to 38, wherein said selector device comprises a selector unit as specified in any one of embodiments 31 to 33.
Embodiment 41: A computer-implemented method of selecting at least two indicator agents for labeling two analytes with two detection agents, comprising
   (a) obtaining data on available indicator agents
   (b) obtaining data on the analyte or analytes; and
   (c) selecting at least two indicator agents based on the data obtained in steps (I) and (II), wherein said selection fulfills at least one condition selected from the list consisting of
      - reduction or prevention of fluorescence spillover,
      - reduction or prevention of overlap of absorption spectra, and
      - reduction or prevention of cross-excitation.
Embodiment 42: Use of on-board conjugation for producing at least two detection agents comprising non-identical indicator agents on a device comprising an analysis unit and a detector.
Embodiment 43: The use of embodiment 42, wherein said analysis unit is an analysis unit as specified in embodiment 28 or 29.
Embodiment 44: The use of embodiment 42 or 43, wherein said detector is a detector as specified in any one of embodiments 28 to 30.
Embodiment 45: The use of any one of embodiments 42 to 44, wherein said device comprises a FACS unit.

All references cited in this specification are herewith incorporated by reference with respect to their entire disclosure content and the disclosure content specifically mentioned in this specification.

### Figure Legends

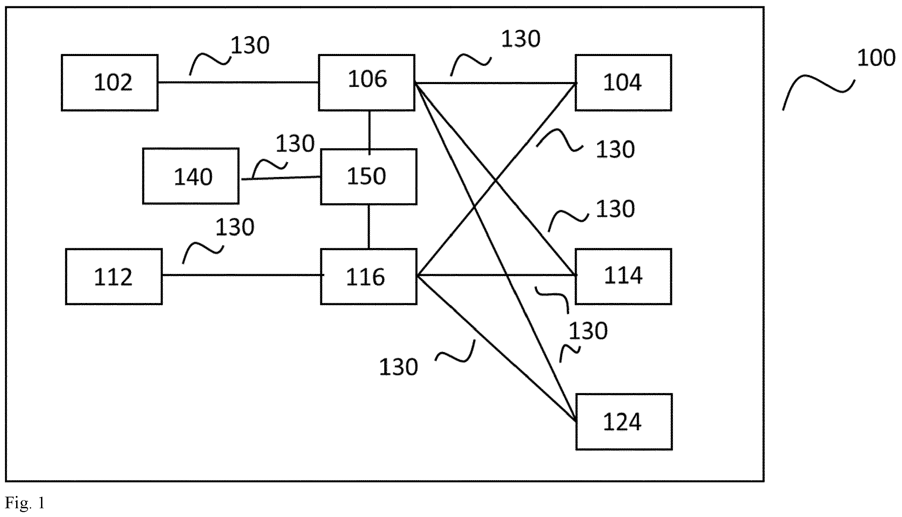

Fig. 1: Schematic depiction of a labeling device 100.

The following Examples shall merely illustrate the invention. They shall not be construed, whatsoever, to limit the scope of the invention.

Referring to Fig. 1, a labeling device 100 of the present invention may comprise a first detection agent reservoir 102 and a second detection agent reservoir 112, a first, second, and a third indicator agent reservoir 104, 114, 124, a first, second, and third reaction vessel 106, 116, and 150, a sample container 140, connected by fluid connections 130 as shown in Fig. 1. As the skilled person understands, the liquid connections may be embodied as tubings, but also as fluid connections provided by a pipetting device, i.e. fluid connections 130 do not have to be permanent.

### Example 1: Flexibility of single indicator agent use

In use, a first detection agent can be transferred from its container 102 to the first reaction vessel 106, and a first indicator agent can be transferred from its container 104 to said first reaction vessel 106, wherein the detection agent and the indicator agent are conjugated; after completion of conjugation, the conjugate is transferred to the second reaction vessel 150, to which further the sample is added from its container 140.

In case the first indicator agent is no longer available, e.g. because it was used up or has reached its use-by date, the method may be performed as described above, but transferring a second indicator agent from its container 114 to the first reaction vessel 106, thus providing a replacement conjugate.

### Example 2: Interference avoidance

Again referring to Fig. 1, a double labelling may be achieved by transferring a first detection agent from its container 102 to the first reactions vessel 106, and a first indicator agent from its container 104 to said first reaction vessel 106, wherein the first detection agent and the first indicator agent are conjugated; after completion of conjugation, the resulting first conjugate is transferred to the second reaction vessel 150. Further, a second detection agent is transferred from its container 112 to the third reaction vessel 116, and a second indicator agent is transferred from its container 114 to said third reaction vessel 116, wherein the second detection agent and the second indicator agent are conjugated; after completion of conjugation, the resulting second conjugate is transferred to the second reaction vessel 150, to which further the sample is added from its container 140, thus achieving a double labeling.

in case it is realized that the combination of first indicator agent and second indicator agent leads to interference, e.g. the second indicator agent may be replaced by a third indicator agent from its container 124, leading to an improved labeling.

### Example 3: Flexibility

As is known to the skilled person, in e.g. TBNK analysis (T cell, B cell, and NK cell analysis), the markers CD3, CD4, CD8, CD45, CD56, CD16, and/or CD19 may be determined, so at least one detection agent for each of said markers is required (Table 1). Also, commonly used indicator agents are e.g. shown in Table 2.

**Table 1: detection agents in TBNK**

| detection agent |
|---|
| |
| anti-CD3 |
| anti-CD4 |
| anti-CD8 |
| anti-C45 |
| anti-CD56 |
| anti-CD16 |
| anti-CD19 |
| |

**Table 2: indicator agents**

| indicator agent |
|---|
| DAPI |
| *fluorescein* |
| *rhodamine* |
| *eosin* |
| CF4055^{™} |
| Brilliant Violet 510^{™} |
| CF488A^{™} |
| APC-Vio770^{™} |
| StarBright Violet 610^{™} |

In order to stock any possible combination of detection agents and indicator agents from Tables 1 and 2, it would be required to stock 7 × 9 = 63 detection agent/indicator agent conjugates separately, with associated effort and cost. According to the claimed subject matter, it is only necessary to stock 7 detection agents and 9 indicator agents, since the required combinations can be conjugated on-board.

### Literature

Barnett et al. (2009), Nat Rev Microbiol 6(11 Suppl):S7
Ouyang et al. (2019), J Int Med Res 47(4):1483
US 9,017,610 B2

### Reference signs:

- 100: labeling device
- 102: first detection agent reservoir
- 104: first indicator agent reservoir
- 106: first reaction vessel
- 112: second detection agent reservoir
- 114: second indicator agent reservoir
- 116: third reaction vessel
- 124: third indicator agent reservoir
- 130: fluid connection, e.g. tubing
- 140: sample container
- 150: second reaction vessel

## Claims

1. A method for labeling an analyte in a sample, said method comprising
(a) providing a detection agent binding to the analyte and conjugating said detection agent with an indicator agent to produce a conjugation product;
(b) contacting said sample with said conjugation product of step (a);
wherein said step (b) is performed directly after completion of step (a) and wherein steps (a) and (b) are performed by the same labeling device.

2. The method of claim 1, wherein performing directly comprises performing step (b) at most 1 day, in an embodiment at most 12h, in a further embodiment at most 6h, in a further embodiment at most 3h, in a further embodiment at most 1h, after completion of step (a).

3. The method of claim 1 or 2, wherein the conjugating step (a) is performed in a first reaction vessel and wherein performing directly comprises contacting the conjugate from step (a) with the sample in a second reaction vessel, wherein the conjugate from step (a) is not contacted to any further vessel before step (b).

4. The method of any one of claims 1 to 3, wherein said detection agent is an antibody or fragment thereof.

5. The method of any one of claims 1 to 4, herein said sample is a biological sample, in an embodiment a sample comprising cells.

6. The method of any one of claims 1 to 5, wherein the coupling between the detection agent and the indicator agent is non-covalent, in an embodiment via an affinity pair.

7. The method of any one of claims 1 to 6, wherein said method comprises an additional step (a1) of providing an additional detection agent binding to an additional analyte, an additional step of conjugating said additional detection agent with an additional indicator agent to produce an additional conjugation product, wherein step (b) comprises contacting said sample with the conjugation product of step (a), and with said additional conjugation product of step (a1), and wherein step (b) is performed directly after completion of at least one of said providing and conjugating steps.

8. The method of any one of claims 1 to 7, wherein said method comprises selecting the indicator agent and additional indicator agent, and optionally any further indicator agent, such that such that interference between said indicator agents is reduced., wherein said interference in an embodiment is selected from the list consisting of fluorescence spillover, overlap of absorption spectra, cross-excitation.

9. The method of any one of claims 1 to 8, wherein said labeling device is a labeling device according to any one of claims 10 to 13.

10. A labeling device comprising
(A) a first container comprising a detection agent, operably connected to a reaction unit;
(B) a second container comprising an indicator agent, operably connected to the reaction unit, and
(C) a labeling unit;
wherein the reaction unit is configured for conjugating said detection agent with the indicator agent to produce a conjugation product, and wherein said labeling unit is configured to contact a sample with the conjugation product,
wherein said labeling device further comprises a processor and tangibly embedded instructions which, when performed on the processor, cause the labeling device to perform the method of any one of claims 1 to 8.

11. The labeling device of claim 10, further comprising an analysis unit comprising a detector.

12. The labeling device of claim 11, wherein said detector comprises a FACS unit or a digital imaging unit.

13. The labeling device of any one of claims 10 to 12 further comprising a selector unit comprising tangibly embedded an algorithm for selecting an indicator agent for conjugation with a detection agent.

14. A system comprising, operably connected,
(i) a labeling device according to any one of claims 10 to 13; and
(ii) a detection device and/or a selector device.

15. Use of on-board conjugation for producing at least two detection agents comprising non-identical indicator agents on a device comprising an analysis unit and a detector.
